Europäisches Patentamt

European Patent Office

Office européen des brevets

(19)

(11) Publication number: **0 127 079**

**A1**

(12) **EUROPEAN PATENT APPLICATION**

(21) Application number: **84105624.5**

(22) Date of filing: **17.05.84**

(51) Int. Cl.³: **C 07 C 85/11**

(30) Priority: **18.05.83 JP 87975/83**

(43) Date of publication of application:
**05.12.84 Bulletin 84/49**

(84) Designated Contracting States:
**CH DE FR GB IT LI**

(71) Applicant: **SUMITOMO CHEMICAL COMPANY, LIMITED**
**15 Kitahama 5-chome Higashi-ku**
**Osaka-shi Osaka-fu(JP)**

(72) Inventor: **Takemoto, Ichiki**
**2-14-7, Mefu**
**Takarazuka Hyogo(JP)**

(72) Inventor: **Fukushima, Masayuki**
**2-14-7, Mefu**
**Takarazuka Hyogo(JP)**

(74) Representative: **Vossius Vossius Tauchner Heunemann**
**Rauh**
**Siebertstrasse 4 P.O. Box 86 07 67**
**D-8000 München 86(DE)**

(54) Process for preparing 2-fluoro-5-nitroaniline.

(57) A process for preparing 2-fluoro-5-nitroaniline from 2,4-dinitrofluorobenzene in a good yield, which comprises treating the latter with powdery iron in the presence of an acid so as to reduce the nitro group at the 2-position selectively.

EP 0 127 079 A1

PROCESS FOR PREPARING 2-FLUORO-5-NITROANILINE

The present invention relates to a process for preparing 2-fluoro-5-nitroaniline. More particularly, it relates to a process for preparing 2-fluoro-5-nitroaniline from 2,4-dinitrofluorobenzene by selective reduction of the nitro group at the 2-position of the latter using iron in the presence of an acid.

Said 2-fluoro-5-nitroaniline is known to be useful as the starting material for production of tetrahydrophthalimides, hydantoins, urazoles, etc., which are per se useful as herbicides (cf. EP-0061741A, EP-0070389A, EP-0075267A, EP-0083055A).

For preparation of 2-fluoro-5-nitroaniline, there is known a process wherein 2,4-dinitrofluorobenzene is subjected to reduction using stannous chloride (cf. J.J. Blanksma et al: Receuil des Travaux Chimiques des Pays-Bas, 65, 329 (1946)). However, said literature describes that the reaction product in this process is a mixture of 2-fluoro-5-nitroaniline as the objective compound and 4-fluoro-3-nitroaniline. In fact, as shown in Comparative Example hereinafter presented, the reaction product in said process is a mixture of 2-fluoro-5-nitroaniline and 4-fluoro-3-nitroaniline in a weight proportion of 4 : 1. In addition, the yield of such mixture is relatively low.

As a result of the extensive study for overcoming the drawback as seen in the conventional process, it has now been found that 2-fluoro-5-nitroaniline as the objective compound can be obtained predominantly in a good yield by treatment of 2,4-dinitrofluorobenzene with iron in the presence of an acid so as to reduce selectively the nitro group at the 2-position.

According to this invention, 2,4-dinitrofluorobenzene is treated with iron in the presence of an acid to reduce the nitro group at the 2-position selectively.

As the iron, there may be used iron powder such as reduced iron or electrolytic iron. Examples of the acid are acetic acid, hydrochloric acid, etc. The amount of the iron and the acid may be respectively from 2.5 to 4 mol and from 0.01 to 1 mol per 1 mol of the starting 2,4-dinitrofluorobenzene. The treatment is usually effected in an inert solvent at a temperature of 60°C to the boiling temperature of the reaction system for a period of 10 minutes to 2 hours. The inert solvent may be chosen, for instance, from aromatic hydrocarbons (e.g. benzene, toluene, xylene), ethers (e.g. diethyl ether, diisopropyl ether, dioxane, tetrahydrofuran, diethylene glycol dimethyl ether), aliphatic acids (e.g. acetic acid, oleic acid), alcohols (e.g. methanol, ethanol, isopropanol, t-butanol, octanol, cyclohexanol, methyl cellosolve, diethylene glycol, glycerol) and water, and their mixtures.

After completion of the reaction, the reaction mixture may be subjected to post-treatment such as concentration, extraction, etc. by a per se conventional procedure so as to recover the produced 2-fluoro-5-nitroaniline. When desired, purification by chromatography, distillation or recrystallization may be applied to the recovered product.

Practical and presently preferred embodiments of the invention are illustratively shown in the following Examples.

Example 1

A suspension of iron powder (electrolytic iron) (8.1 g) in acetic acid (100 ml) was heated under reflux, and a solution of 2,4-dinitrofluorobenzene (9 g) in acetic acid (30 ml) was added thereto. The resultant mixture was refluxed for 10 minutes, poured into ice-water and extracted with ethyl acetate. The extracts were washed with a saturated sodium bicarbonate solution, water and a saturated sodium chloride solution in order, dried over anhydrous magnesium sulfate and concentrated. The crude crystals were recrystallized from ethanol to give 5.3 g of 2-fluoro-5-nitroaniline as orange crystals. Yield, 70 %. M.P., 97 - 98°C. The product was identified with the authentic sample in melting point, NMR data and GLC (QF-1.3 m, 130°C, $N_2$ 1.2 kg/cm²).

The weight proportion of 2-fluoro-5-nitroaniline and 4-fluoro-3-nitroaniline in said crude crystals as determined by GLC under the condisition as above was 93 : 7.

Example 2

A suspension of iron powder (electrolytic iron) (8.1 g) in water (100 ml) and conc. hydrochloric acid (2 ml) was heated under reflux, and 2,4-dinitrofluorobenzene (9 g) was added thereto. The resultant mixture was refluxed for 10 minutes and, after cooling, extracted with ethyl acetate. The extracts were subjected to silica gel column chromatography using a mixture of ethyl acetate and n-hexane as an eluting solvent to give 2 g of 2-fluoro-5-nitroaniline as yellow crystals. Yield, 49 %. Besides, 4.1 g of 2,4-dinitrofluorobenzene were recovered. Recovery rate, 46 %.

Example 3

A suspension of iron powder (reduced iron) (10.8 g) in water (100 ml) and conc. hydrochloric acid (0.06 g) was heated under reflux, and 2,4-dinitrofluorobenzene (9 g) was added thereto. The resultant mixture was refluxed for 2 hours and, after cooling, extracted with ethyl acetate. The extracts were subjected to silica gel column chromatography using a mixture of ethyl acetate and n-hexane as an eluting solvent to give 2.5 g of 2-fluoro-5-nitroaniline as yellow crystals. Yield, 50 %. Besides, 3.0 g of 2,4-dinitrofluorobenzene were recovered. Recovery rate, 33 %.

Example 4

To a suspension of iron powder (electrolytic iron) (108 g) in acetic acid (1000 g), a solution of 2,4-dinitrofluorobenzene (130 g) in acetic acid (200 g) was added dropwise at a temperature of 60 to 110°C, followed by stirring at a temperature of 110 to 138°C for 1.5 hours. The reaction mixture was extracted with ethyl acetate, and the extracts were concentrated to give 86 g of 2-fluoro-5-nitroaniline. Yield, 79 %.

Comparative Example 1 (according to the J.J.

Blanksma's method)

To a solution of stannous chloride (30 g) in 10 % hydrochloric acid (100 ml) kept at a temperature below 10°C by cooling with ice, a solution of 2,4-dinitrofluorobenzene (10 g) in a mixture of ethanol (50 ml) and water (100 ml) was added dropwise, and the resultant mixture was stirred at room temperature for 2 hours. The reaction mixture was made alkaline with an aqueous sodium hydroxide solution under cooling with ice and extracted with ethyl acetate. The extracts were washed with water and a saturated sodium chloride solution, dried over anhydrous magnesium sulfate and concentrated. The oily product was purified by silica gel column chromatography using a mixture of ethyl acetate and n-hexane as an eluting solvent to give 2-fluoro-5-nitroaniline (2 g; yield, 24 %) and 4-fluoro-3-nitroaniline (0.5 g; yield, 6 %).

What is claimed is:

1. A process for preparing 2-fluoro-5-nitroaniline, which comprises treating 2,4-dinitrofluorobenzene with iron in the presence of an acid so as to reduce the nitro group at the 2-position.

2. The process according to claim 1, wherein the iron is reduced iron or electrolytic iron.

3. The process according to claim 1, wherein the acid is acetic acid or hydrochloric acid.

4. The process according to claim 1, wherein the molar proportion of the iron to the 2,4-dinitrofluorobenzene is from 2.5 to 4.

5. The process according to claim 1, wherein the molar proportion of the acid to the 2,4-dinitrofluorobenzene is from 0.01 to 1.

European Patent Office

**EUROPEAN SEARCH REPORT**

Application number

EP 84 10 5624

## DOCUMENTS CONSIDERED TO BE RELEVANT

| Category | Citation of document with indication, where appropriate, of relevant passages | Relevant to claim | CLASSIFICATION OF THE APPLICATION (Int. Cl ³) |
|---|---|---|---|
| A | DE-C- 67 018 (A. WÜLFING) <br> * Page 2 * | 1-5 | C 07 C 85/11 |
| A | G. SCHIEMANN et al.: "Chemie und Technologie cyclischer Fluorverbindungen", 1969, pages 64-66,398,399,403,414,416,427,453, Ferdinand Enke Verlag, Stuttgart, DE; <br> * Page 65 * | 1-5 | |
| D,A | REC. TRAV. CHIM. PAYS-BAS, vol. 65, 1946, pages 329-332, The Hague, NL; J.J. BLANKSMA et al.: "On the sweet taste of the 1-halogeno-2-amino-4-nitrobenzenes" <br> * Page 329 * | 1-5 | |
| A | CHEMISTRY AND INDUSTRY, 15th February 1969, pages 198,199; M. BIL: "Improved preparation of 4-fluoro-2-nitroaniline, 2-fluoro-5-nitroaniline and 4-nitro-1,2-phenylene diamine" <br> * Page 199, preparation of III * | 1 | TECHNICAL FIELDS SEARCHED (Int. Cl ³) <br><br> C 07 C 85/00 |

The present search report has been drawn up for all claims

| Place of search | Date of completion of the search | Examiner |
|---|---|---|
| THE HAGUE | 16-08-1984 | PAUWELS G.R.A. |